(19) Europäisches Patentamt
European Patent Office
Office européen des brevets

(11) Publication number: 0 103 529 A1

# (12) EUROPEAN PATENT APPLICATION

(21) Application number: 83401799.8

(22) Date of filing: 14.09.83

(51) Int. Cl.³: C 07 C 69/732, C 07 C 67/343

(30) Priority: 15.09.82 US 418575

(43) Date of publication of application: 21.03.84 Bulletin 84/12

(84) Designated Contracting States: BE CH DE FR GB LI

(71) Applicant: ETHYL CORPORATION, Ethyl Tower 451 Florida Boulevard, Baton Rouge Louisiana 70801 (US)

(72) Inventor: Everly, Charles Ray, 1431 Munal Drive, Baton Rouge Louisiana 70816 (US)
Inventor: Roper, Jerry Monroe, 5821 Burgundy Drive, Baton Rouge Louisiana 70806 (US)

(74) Representative: Rinuy, Guy et al, Cabinet Rinuy et Santarelli 14, Avenue de la Grande Armée, F-75017 Paris (FR)

## (54) Novel substituted benzylated malonic acid esters and process therefor.

(57) The present invention deals with novel substituted benzylated malonic acid esters and a process for their preparation.

Esters of 3,5-dihydrocarbyl-4-hydroxybenzyl-malonic acid are prepared by reacting an N,N-dihydrocarbyl-2,6-dihydrocarbyl-4-aminomethylphenol with an ester of a 1,3-dicarboxylic acid in the presence of an alkali or an alkaline earth metal hydride.

The resulting substituted benzylated malonic acid esters are useful as antioxidants into polymers, latices, liquid hydrocarbon fuels and lubricating oils.

EP 0 103 529 A1

# NOVEL SUBSTITUTED BENZYLATED MALONIC ACID ESTERS AND PROCESS THEREFOR

This invention relates to 3,5-dihydrocarbyl-4-hydroxybenzylmalonic acid esters and the preparation and uses thereof as antioxidants for oxidizable organic materials when such materials are exposed to oxidative degradative conditions.

The materials of the invention are prepared by reacting an N,N-dihydrocarbyl-2,6-dihydrocarbyl-4-aminomethylphenol with an ester of a 1,3-dicarboxylic acid in the presence of an alkali or an alkaline earth metal hydride. Thus, in one embodiment of the invention there is provided a novel process for the preparation of 3,5-dihydrocarbyl-4-hydroxybenzylmalonic acid esters which comprises reacting an N,N-dihydrocarbyl-2,6-dihydro-carbyl-4-aminomethylphenol with an ester of a 1,3-dicarboxylic acid in the presence of an alkali or an alkaline earth metal hydride.

The process can be illustrated schematically by the following structural formulas. Compounds having the general formula:

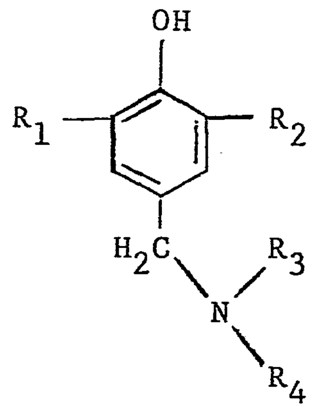

(I)

are reacted with compounds having the general formula:

$$R_5O\overset{O}{\overset{\|}{C}}CH_2\overset{O}{\overset{\|}{C}}OR_6 \qquad \text{(II)}$$

in the presence of an alkali or an alkaline earth metal hydride to yield a benzylated malonic acid ester having the structural formula:

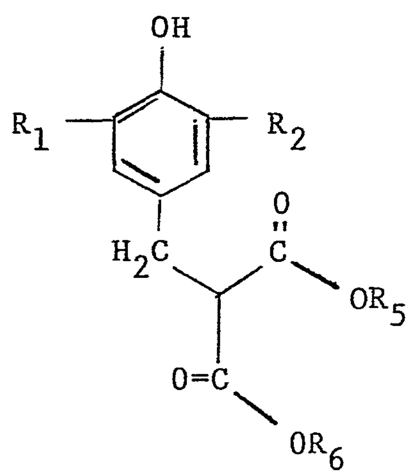

(III)

In the structural formulas above, $R_1$ and $R_2$ are the same or different and are hydrogen or hydrocarbyl radicals, preferably alkyl, aralkyl or cycloalkyl radicals having from 1 up to 40 carbon atoms, and preferably from 3 to 8 atoms, preferably at least one of which is branched on the alpha-carbon atom, with the provision that at least one of $R_1$ or $R_2$ must be other than hydrogen; $R_3$ and $R_4$ are the same or different and are linear, branched or unbranched alkyl, aralkyl or cycloalkyl radicals having from 1 to 20 carbon atoms; $R_5$ and $R_6$ are the same or different and can be hydrogen or linear or branched alkyl radicals having from 1 to 20 carbon atoms with the provision that at least one of $R_5$ or $R_6$ must be other than hydrogen.

Thus, in another embodiment of the present invention there is provided a process for the preparation of 3,5-dihydrocarbyl-4-hydroxy-benzylmalonic acid esters having the general formula:

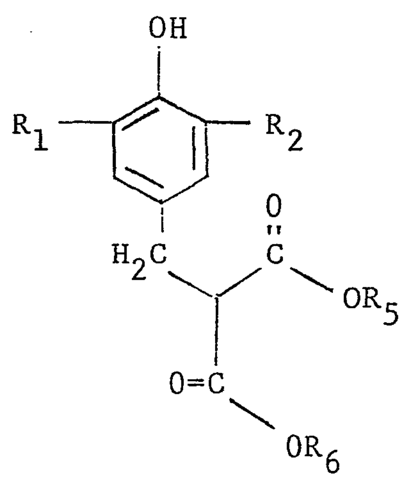

(III)

which comprises reacting an N,N-dihydrocarbyl-2,6-dihydrocarbyl-4-aminomethylphenol of the general formula:

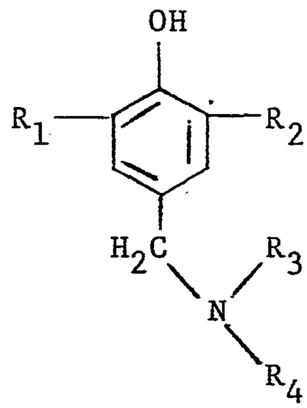

(I)

with an ester of a 1,3-dicarboxylic acid of the general formula:

$$R_5O\overset{O}{\overset{\|}{C}}CH_2\overset{O}{\overset{\|}{C}}OR_6 \qquad (II)$$

in the presence of an alkali or an alkaline earth metal hydride wherein in the structural formulas above $R_1$ and $R_2$ are the same or different and are hydrogen or hydrocarbyl radicals having from 1 to 40 carbon atoms, preferably at least one of which is branched on the alpha-carbon atom, with the provision that at least one of $R_1$ or $R_2$ must be other than hydrogen; $R_3$ and $R_4$ are the same or different and are linear, branched or unbranched alkyl, aralkyl or cycloalkyl radicals having from 1 to 20 carbon atoms; $R_5$ and $R_6$ are the same or different and are hydrogen or linear or branched alkyl radicals having from 1 to 20

carbon atoms with the provision that at least one of $R_5$ or $R_6$ must be other than hydrogen.

Representative examples of radicals described above are secondary radicals such as secondary butyl, secondary amyl, secondary octyl; tertiary radicals such as tertiary butyl, tertiary hexyl and tertiary decyl; alkyl radicals such as methyl, ethyl, propyl, butyl, nonyl, decyl, tetradecyl, hexadecyl, nonadecyl; aralkyl radicals such as methyl phenyl and pentyl phenyl, and cycloalkyl radicals such as cyclopentyl, cyclohexyl and cycloheptyl radicals.

Representative examples of the Formula I compounds are

N,N-dimethyl-2,6-di-t-butyl-4-aminomethylphenol,

N,N-dimethyl-2-methyl-6-isopropyl-4-aminomethyl-phenol,

N,N-dimethyl-2-methyl-6-t-butyl-4-aminomethylphenol,

N,N-dimethyl-2,6-diisopropyl-4-aminomethylphenol,

N,N-dimethyl-2-sec-butyl-4-aminomethylphenol,

N,N-dimethyl-2-isopropyl-4-aminomethylphenol,

N,N-dimethyl-2-t-butyl-4-aminomethylphenol,

N,N-diethyl-2,6-di-t-butyl-4-aminomethylphenol,

N,N-dioctyl-2,6-di-t-butyl-4-aminomethylphenol,

N,N-dioctyl-2-ethyl-6-t-butyl-4-aminomethylphenol,

N,N-dioctyl-2,6-diheptyl-4-aminomethylphenol,

N,N-dioctyl-2-ethyl-6-methyl-4-aminomethylphenol,

N,N-dioctyl,2-t-butyl-6-heptyl-4-aminomethylphenol,

N-ethyl-N-methyl-2,6-di-t-butyl-4-aminomethylphenol, and

N-octyl-N-methyl-2-methyl-6-ethyl-4-aminomethylphenol.

Representative examples of Formula II esters of 1,3-dicarboxylic acid compounds are

malonic acid, dimethyl ester,

malonic acid, diethyl ester,

malonic acid, diisopropyl ester,

malonic acid, di-n-hexyl ester,

malonic acid, dioctyl ester,

malonic acid, didodecyl ester,

malonic acid, ethyl, methyl diester,

malonic acid, ethyl, isopropyl diester,

malonic acid, n-butyl, ethyl diester,

malonic acid, n-butyl, dodecyl diester,

malonic acid, octyl, ethyl diester,

malonic acid, ethyl monoester,

malonic acid, n-propyl monoester,

malonic acid, n-butyl monoester,

malonic acid, n-hexyl monoester,

malonic acid, octyl monoester,

malonic acid, dodecyl monoester, and the like.

Representative examples of Formula III benzylated malonic acid esters, functioning as antioxidants, are

3,5-di-t-butyl-4-hydroxybenzylmalonic acid, dimethyl ester,

3,5-di-t-butyl-4-hydroxybenzylmalonic acid, diethyl ester,

3,5-di-t-butyl-4-hydroxybenzylmalonic acid, diisopropyl ester,

3,5-di-t-butyl-4-hydroxybenzylmalonic acid, di-n-hexyl ester,

3,5-di-t-butyl-4-hydroxybenzylmalonic acid, dioctyl ester,

3,5-di-t-butyl-4-hydroxybenzylmalonic acid, didodecyl ester,

3,5-di-t-butyl-4-hydroxybenzylmalonic acid, ethyl, methyl diester,

3,5-di-t-butyl-4-hydroxybenzylmalonic acid, ethyl, isopropyl diester,

3,5-di-t-butyl-4-hydroxybenzylmalonic acid, n-butyl, ethyl diester,

3,5-di-t-butyl-4-hydroxybenzylmalonic acid, n-butyl, dodecyl diester,

3,5-di-t-butyl-4-hydroxybenzylmalonic acid, octyl, methyl diester,

3,5-di-t-butyl-4-hydroxybenzylmalonic acid, octyl, ethyl diester,

3,5-di-t-butyl-4-hydroxybenzylmalonic acid, ethyl monoester,

3,5-di-t-butyl-4-hydroxybenzylmalonic acid, n-propyl monoester,

3,5-di-t-butyl-4-hydroxybenzylmalonic acid, n-butyl monoester,

3,5-di-t-butyl-4-hydroxybenzylmalonic acid, n-hexyl monoester,

3,5-di-t-butyl-4-hydroxybenzylmalonic acid, octyl monoester,

3,5-di-t-butyl-4-hydroxybenzylmalonic acid, dodecyl monoester,

3-ethyl-5-ethyl-4-hydroxybenzylmalonic acid, dioctyl ester,

3-n-butyl-5-octyl-4-hydroxybenzylmalonic acid, ethyl, methyl diester,

3-ethyl-5-methyl-4-hydroxybenzylmalonic acid, ethyl monoester,

3,5-dioctyl-4-hydroxybenzylmalonic acid, octyl monoester, and the like.

BAD ORIGINAL

In general, any of the alkali or alkaline earth metal hydrides may be used in the practice of the present process. These include sodium hydride, potassium hydride, lithium hydride, magnesium hydride, calcium hydride, and the like. Sodium hydride is preferred.

The process of the invention is carried out by reacting the benzylamine starting material with at least 1 molar equivalent of the malonic acid ester reactant although an excess of ester reactant can be used. A preferred range of malonic acid ester reactant to benzylamine reactant is from 1 to 10 moles of ester per mole of benzylamine.

At least 1 mole of hydride per mole of benzylamine reactant should be used in the process of the invention, althoughan amount of hydride up to 50 moles of hydride per mole of benzylamine reactant can be used, if desired.

The reaction is advantageously conducted at a temperature of from 50$^{o}$C. to 500$^{o}$C. While lower temperatures can be used, the reaction rates are generally correspondingly lower. Temperatures above 500$^{o}$C. can be used, but excessive decomposition of the reaction components can occur. Reflux temperature at atmospheric pressure is effective and preferred.

Typically, the reaction can be conducted at atmospheric pressure. However, higher pressures up to about 68.9 x $10^5$ Pa of manometric pressure (1000 psig) may be used, if desired.

The use of a solvent for the reaction mixture is not generally required, especially if an excess of malonic acid ester reactant is used. However, if desired, a solvent which is inert under the reaction conditions, i.e., those solvents which do not enter into the reaction, may be added to the reaction vessel. Useful solvents comprise aprotic solvents which include ethers such as diethyl ether, dibutyl ether, 1-ethoxyhexane, tetrahydrofuran, 1,4-dioxane, 1,3-dioxolane, diglyme, 1,2-diethoxyethane, and tertiary amines such as pyridine, N-ethylpiperidine, triethylamine, tributylamine, N,N-diphenyl-N-methylamine, N,N-dimethylalanine, etc. Especially useful solvents are dipolar aprotic solvents such as dimethyl sulfoxide, N,N-dimethylformamide, N,N-dimethylacetamide, dimethyl sulfone, tetramethylene sulfone, N-methylpyrrolidinone, acetonitrile and like materials. Other solvents which are inert under the reaction conditions may be used: for example, low boiling hydrocarbons, halogenated hydrocarbons, examples of which are benzene, toluene, tetrachloroethane, the chlorinated benzenes, the chlorinated toluenes, etc., and lower alkanols having up to about 6 carbon atoms. These include, methanol, ethanol,

n-propanol, isopropyl alcohol, n-butanol, sec-butyl alcohol, t-butyl alcohol, n-pentanol, isopentyl alcohol, n-hexanol and isohexyl alcohol.

The amount of solvent can be expressed as a volume ratio of solvent to benzylamine reactant. Suitable volume ratios of solvent to benzylamine reactant can be from 0/1 to 500/1 and preferably from 1/1 to 300/1.

The mode of addition in the process is not particularly critical. Accordingly, it is convenient to add the benzylamine reactant to a mixture of the other materials, add the malonic acid ester compound to a mixture of the other materials, add the reactants to a mixture of the benzylamine and solvent, introduce all ingredients simultaneously into the reaction zone, or the like.

The process should be carried out for the time sufficient to convert substantially all of the benzylamine reactant to the corresponding benzylated malonic acid ester. The length of time for optimum yield will depend primarily upon the reaction temperature and the particular solvent, if any, used in the reaction. In general, excellent yields of the benzylated malonic acid ester are obtained in from two to twenty-four hours.

Although not required, the process can be conducted in a substantially anhydrous reaction system, and accordingly, the components of the reaction system are brought together and maintained under a substantially dry, inert atmosphere. By "substantially anhydrous" is meant a reaction system wherein the total amount of water present is no more than about 5 percent by weight, based on the reaction mixture. When the amount of water in the system exceeds this, both reaction rate and yield of product decrease.

The process may readily be conducted in a batchwise, semi-batch or continuous manner and in conventional equipment.

Optionally, metal halides such as the alkali metal and alkaline earth metal halides, for example, magnesium chloride, calcium chloride, sodium chloride, barium chloride, potassium chloride and the like, can be introduced into the reaction mixture, preferably in an amount from .01 to 10 mole percent of metal halide per mole of benzylamine reactant. It has been found that under certain reaction conditions, the presence of a metal halide in the reaction system appears to have a yield-enhancing effect on the formation of the desired 3,5-dihydrocarbyl-4-hydroxybenzylmalonic acid ester product of the instant process. The process of the invention when run continuously can be illustrated schematically by the equation shown below. $R_1$, $R_2$, $R_3$, $R_4$, $R_5$, and $R_6$ are the same radicals as described and exemplified above.

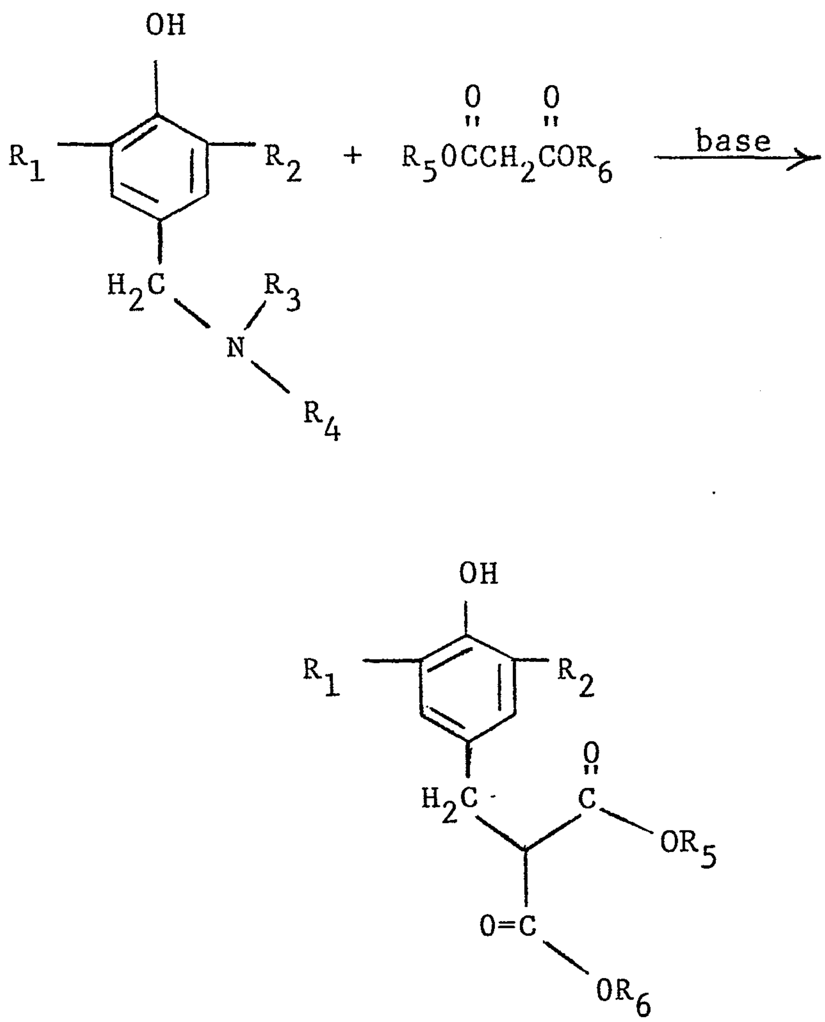


Under the reaction conditions, the benzylamine reactant is fragmented to yield a quinone methide intermediate which undergoes nucleophilic attack by the malonic acid ester reactant to form the desired benzylated malonic acid ester product. Also, a secondary amine is eliminated from the benzylamine starting

reactant during the course of the reaction. Some bis(hydroxyphenyl)methane by-product may be formed by the process.

The benzylated malonic acid ester product is easily separated from the reaction mixture by such means as distillation, extraction, crystallization and other methods obvious to those skilled in the chemical processing art.

The benzylated malonic acid ester products prepared by the process of this invention have antioxidant properties and are capable of stabilizing polymers normally subject to oxidative degradation when incorporated into the polymers using conventional techniques such as by addition to polymer lattices; or by addition to solid polymers on a mill or in a Banbury. Further, the novel compounds of this invention are effective antioxidants in both unleaded and leaded gasolines made from a wide variety of base stocks and for engine and industrial oils which are derived from crude petroleum or produced synthetically.

Thus, in another embodiment of the present invention there is provided a liquid hydrocarbon fuel of the gasoline boiling range for spark ignition internal combustion engines normally susceptible to deterioration in the presence of oxygen containing in an amount sufficient to inhibit such deterioration, a compound of the general formula:

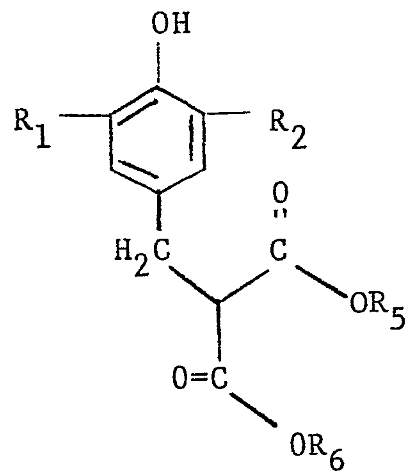

(III)

wherein $R_1$ and $R_2$ are the same or different and are hydrogen or hydrocarbyl radicals, preferably alkyl, aralkyl or cycloalkyl radicals, having from 1 to 40 carbon atoms, and preferably from 3 to 8 carbon atoms, at least one of which is branched on the alpha-carbon atom, with the provision that at least one of $R_1$ or $R_2$ must be other than hydrogen and $R_5$ and $R_6$ are the same or different and are hydrogen or linear or branched alkyl radicals having from 1 to 20 carbon atoms with the provision that at least one of $R_5$ or $R_6$ must be other than hydrogen.

A still further embodiment of the present invention is lubricating oil normally susceptible to oxidative deterioration containing a small antioxidant quantity of a compound of the general formula:

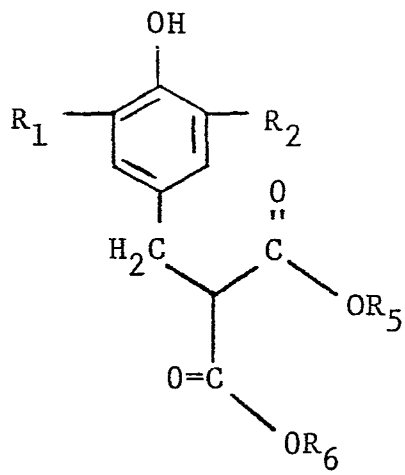

(III)

wherein $R_1$ and $R_2$ are the same or different and are hydrogen or hydrocarbyl radicals, preferably alkyl, aralkyl or cycloalkyl radicals, having from 1 to 40 carbon atoms, and preferably from 3 to 8 carbon atoms, at least one of which is branched on the alpha-carbon atom, with the provision that at least one of $R_1$ or $R_2$ must be other than hydrogen and $R_5$ and $R_6$ are the same or different and are hydrogen or linear or branched alkyl radicals having from 1 to 20 carbon atoms with the provision that at least one of $R_5$ or $R_6$ must be other than hydrogen.

The practice of this invention will be still further apparent by the following illustrative examples.

Example I

To a dimethylformamide solution (20 mls) of N,N-dimethyl-2,6-di-t-butyl-4-aminomethylphenol (2.63 g; 10 mmols) containing magnesium chloride (0.94 g, 10 mmols),

a dimethylformamide solution (10 mmols) of diethyl sodiomalonate (generated by treating a dimethylformamide solution of 1.9 g; 12 mmols diethyl malonate with 0.34 g; 14 mmol oil-free sodium hydride) was added with stirring under a nitrogen atmosphere. The reaction mixture was heated to a temperature between 105°C and 110°C for 16 hours and then poured into cold 2N hydrochloric acid (100 mls). The aqueous reaction slurry was extracted with diethyl ether (3 x 30 mls). The combined organic extract was dried ($MgSO_4$) and concentrated to give 3.36 g; 89% by VPC of 3,5-di-t-butyl-4-hydroxybenzylmalonic acid, diethyl ester as a crude yellow oil.

In a manner similar to Example I above, a number of experiments were carried out varying the temperature, reaction time, solvent and amounts of reactants. The results were analyzed by vapor phase chromatography and are shown in the Table below.

BAD ORIGINAL

TABLE

| Run No. | Reactant[1] (g;mmol) | Diethyl malonate (g;mmol) | Sodium Hydride (g;mmols) | Solvent mls | Time (hr.) | Temp. (°) | % Yield[3] |
|---|---|---|---|---|---|---|---|
| 2 | 2.63; 10 | 1.6; 10 | 0.36; 15 | DMF[2] - 15 | 2 | 120 | 15% |
| 3 | 2.63; 10 | 2.4; 15 | 0.55; 23 | Ethanol - 25 | 3.5 | Reflux | 17% |
| 4 | 13.15; 50 | 8.8; 55 | 1.37; 57 | DMF - 200 | 16 | 110 | 31%[4] |
| 5 | 7.89; 30 | 5.6; 35 | 0.84; 35 | DMF - 150 | 16 | 110 | 44% |

1 N,N-dimethyl,2,6-di-t-butyl-4-aminomethylphenol

2 Dimethylformamide

3 of 3,5-di-t-butyl-4-hydroxybenzylmalonic acid, diethyl ester

4 2.35 grams (25 mmols) $MgCl_2$ added to reaction mixture at beginning of run

CLAIMS:

1. A 3,5-dihydrocarbyl-4-hydroxybenzylmalonic acid ester compound having the general structural formula

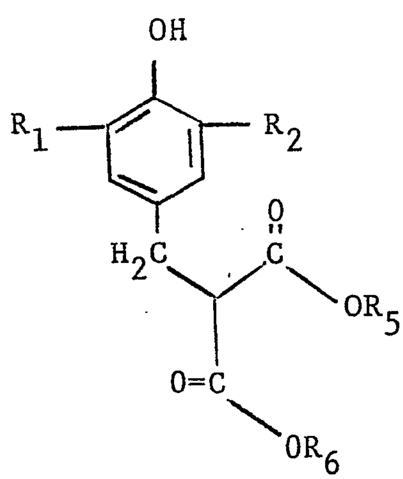

(III)

wherein $R_1$ and $R_2$ are the same or different and are hydrogen or hydrocarbon radicals selected from the group consisting of alkyl, aralkyl or cycloalkyl radicals having from 1 to 40 carbon atoms with the provision that at least one of $R_1$ or $R_2$ must be other than hydrogen and $R_5$ and $R_6$ are the same or different and are hydrogen or linear or branched alkyl radicals having from 1 to 20 carbon atoms with the provision that at least one of $R_5$ or $R_6$ must be other than hydrogen.

2. The compounds of Claim 1 which is selected from the group consisting of

3,5-di-t-butyl-4-hydroxybenzylmalonic acid, dimethyl ester,

3,5-di-t-butyl-4-hydroxybenzylmalonic acid, diethyl ester,

3,5-di-t-butyl-4-hydroxybenzylmalonic acid, diisopropyl ester,

3,5-di-t-butyl-4-hydroxybenzylmalonic acid, di-n-hexyl ester, and

3,5-di-t-butyl-4-hydroxybenzylmalonic acid, dioctyl ester.

3. A process for the preparation of 3,5-dihydrocarbyl-4-hydroxybenzylmalonic acid esters of Claim 1 having the general structural formula

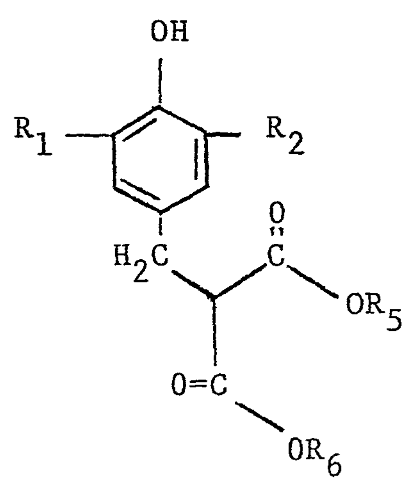

(III)

which comprises reacting an N,N-dihydrocarbyl-2,6-dihydrocarbyl-4-aminomethylphenol of the general structural formula

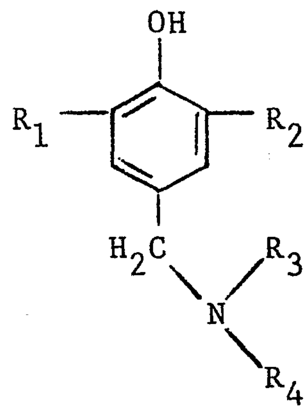

(I)

with an ester of a 1,3-dicarboxylic acid of the general structural formula

$$R_5O\overset{O}{\overset{\|}{C}}CH_2\overset{O}{\overset{\|}{C}}OR_6 \qquad (II)$$

in the presence of an alkali metal hydride or an alkaline earth metal hydride wherein $R_1$ and $R_2$ are the same or different and are hydrogen or hydrocarbyl radicals having from 1 to 40 carbon atoms with the provision that at least one of $R_1$ or $R_2$ must be other than hydrogen, $R_3$ and $R_4$ are the same or different and are linear, branched or unbranched alkyl, aralkyl, or cycloalkyl radicals having from 1 to 20 carbon atoms and $R_5$ and $R_6$ are the same or different and are hydrogen or linear or branched alkyl radicals having from 1 to 20 carbon atoms with the provision that at least one of $R_5$ or $R_6$ must be other than hydrogen.

4. The process of Claim 3 in which the molar ratio of malonic acid ester reactant to benzylamine reactant is from 1-10 moles of malonic acid ester per mole of benzylamine.

5. The process of Claim 3 in which said reaction is conducted in the presence of an added metal halide which is an alkali metal halide or an alkaline earth metal halide.

6. The process of Claim 5 in which the amount of said metal halide is from .01 to 10 mole percent of metal halide per mole of benzylamine reactant.

7. The process of Claim 3 in which said reaction is carried out at a temperature of from 50°C to about 500°C and under pressure in the range of from about atmospheric up to about $68.9 \times 10^5$ Pa of manometric pressure (1000 psig).

8. The process of Claim 3 in which said reaction is carried out in the presence of a solvent which is inert under the reaction conditions.

9. The process of Claim 8 in which said aprotic solvent is a dipolar aprotic solvent selected from dimethyl sulfoxide, N,N-dimethylformamide, N,N-dimethylacetamide, dimethyl sulfone, tetramethylene sulfone, N-methylpyrrolidinone and acetonitrile.

10. The process of Claim 3 in which the reaction is carried out under a substantially dry inert atmosphere.

0103529

Application number

European Patent Office

# EUROPEAN SEARCH REPORT

EP 83 40 1799

| Category | Citation of document with indication, where appropriate, of relevant passages | Relevant to claim | CLASSIFICATION OF THE APPLICATION (Int. Cl. 3) |
|---|---|---|---|
| X | US-A-4 081 475 (JOHN D. SPIVACK) * Column 2, lines 15-54; column 6, example 3; columns 6,7, example 5; columns 9,10; claim 1 * | 1,3,4, 7-9 | C 07 C 69/732<br>C 07 C 67/343 |
| X | US-A-3 878 237 (H. EGGENSPERGER) * Column 1, lines 11-51; columns 2-4, example 1, table I * | 1,3,4, 7,8 | |
| | | | TECHNICAL FIELDS SEARCHED (Int. Cl. 3) |
| | | | C 07 C 69/00<br>C 07 C 67/00 |

The present search report has been drawn up for all claims

| Place of search | Date of completion of the search | Examiner |
|---|---|---|
| THE HAGUE | 05-01-1984 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82